# EUROPEAN PATENT APPLICATION

(11) **EP 4 302 763 A1**
(43) Date of publication of application: **10.01.2024**
(21) Application number: 22762608.2
(22) Date of filing: 03.03.2022
(51) Int. Cl.: A61K 31/565, C07D 403/14, A61P 35/00

(54) **COMBINED PHARMACEUTICAL COMPOSITION CONTAINING CDK4/6 INHIBITOR AND USE THEREOF**

(30) Priority: 03.03.2021 CN 202110236331
(71) Applicant: CHIA TAI TIANQING PHARMACEUTICAL GROUP CO., LTD., Lianyungang, Jiangsu 222062 (CN)
(72) Inventor: FENG, Fan, Lianyungang, Jiangsu 222062 (CN); YU, Ding, Lianyungang, Jiangsu 222062 (CN); WANG, Xunqiang, Lianyungang, Jiangsu 222062 (CN); ZHANG, Xiquan, Lianyungang, Jiangsu 222062 (CN); BAI, Yanfeng, Lianyungang, Jiangsu 222062 (CN); YANG, Chaoqiang, Lianyungang, Jiangsu 222062 (CN); ZHANG, Yuying, Lianyungang, Jiangsu 222062 (CN)
(74) Representative: Thoma, Michael
(86) International application number: PCT/CN2022/079100
(87) International publication number: WO 2022/184146

(57) **Abstract**

The present disclosure relates to a combined pharmaceutical composition containing a CDK4/6 inhibitor and a use thereof, and specifically relates to a combined pharmaceutical composition comprising a compound of formula (I) or a pharmaceutically acceptable salt thereof and fulvestrant. The present disclosure further relates to a use of the combined pharmaceutical composition in treating breast cancer. The combined pharmaceutical composition of the present disclosure produces better therapeutic effects in reducing tumor growth or even eliminating tumors.

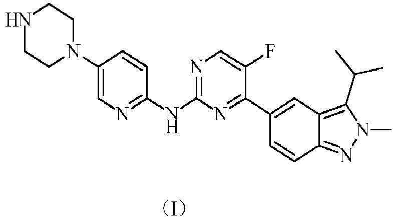

## Description

### CROSS-REFERENCE TO RELATED APPLICATIONS

The present disclosure claims the benefit and priority to the Chinese Patent Application No. 202110236331.5 filed with National Intellectual Property Administration, PRC on March 3, 2021, which is incorporated herein by reference in its entirety.

### TECHNICAL FIELD

The present disclosure belongs to the technical field of medicines, and relates to a combined pharmaceutical composition of a CDK4/6 inhibitor and use thereof in treating breast cancer.

### BACKGROUND

Cyclin-dependent kinase (CDK) 4/6 is a key regulator of cell cycle and is capable of triggering the cell cycle from the growth phase (G1 phase) to the DNA replication phase (S1 phase). In the process of cell proliferation, the complex formed by CDK4/6 and cyclin D can phosphorylate the retinoblastoma protein (Rb). Once the tumor suppressor protein Rb is phosphorylated, it can release transcription factor E2F to which it tightly binds in the unphosphorylated state. E2F activates further transcription to promote the cell cycle through the restriction point (R point) from G1 phase to S phase. Therefore, the inhibition of CDK4/6 and thus its inability to form cyclin D-CDK4/6 complex, can block the progression of cell cycle from G1 phase to S phase, thereby achieving the purpose of inhibiting tumor proliferation. A substituted 2-hydrogen-pyrazole derivative as a selective CDK4/6 inhibitor is disclosed in WO2016141881, and a compound of formula (I) with the following structure is also specifically disclosed:

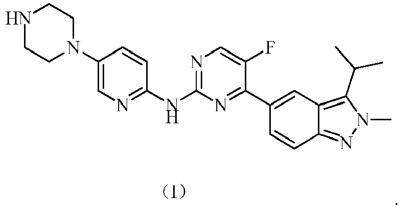

The chemical name of fulvestrant is 7-α-[9-(4,4,5,5,5-pentafluoropentylsulfinyl)nonyl]estra-1,3,5-(10)-triene-3,17-β-diol. Fulvestrant has a relative molecular mass of 606.77, is white or off-white powder, and is insoluble in water. Fulvestrant is a novel estrogen receptor antagonist-estrogen receptor downregulator anti-breast cancer therapeutic agent, and can bind to an estrogen receptor at the cellular level and block and degrade the estrogen receptor, so that the growth of tumor cells under the action of estrogen is blocked.

The worldwide cancer report data shows about 1.7 million new cases of breast cancer and about 0.5 million deaths worldwide in 2012, which respectively account for 25% of all new cancers and 15% of all cancer deaths in women and both rank first. In new breast cancer cases each year, 3%-10% of women have distant metastasis at the time of diagnosis. 30%-40% of patients with early breast cancer may develop advanced breast cancer with a 5-year survival rate of about 20%.

Patients with advanced breast cancer (ABC) are special in the selection and efficacy of treatment regimens. Currently, there is still a lack of a recognized standard treatment regimen. The overall median survival time for advanced breast cancer is 2-3 years, varying between different molecular subtypes. For ABC patients positive for human epidermal growth factor receptor 2 (HER2), anti-HER2 treatment may alter the natural course of HER2 positive ABC patients and significantly prolong survival time; however, for triple-negative ABC patients, the overall prognosis has not improved significantly. Hormone receptor positive (HR+) breast cancer accounts for about 65%-75% of breast cancer, and endocrine therapy is the first choice for hormone receptor positive (HR+) metastatic breast cancer due to its equivalent efficacy and decreased toxic and side effects compared with chemotherapy.

Although endocrine therapy is the main treatment regimen of hormone receptor positive breast cancer, about 30% of hormone receptor positive breast cancer has primary drug resistance to endocrine therapy, and almost all patients have secondary drug resistance in follow-up treatment. Therefore, how to overcome the endocrine therapy resistance is a problem to be solved in the field of breast cancer treatment.

### SUMMARY

In one aspect, the present disclosure provides a combined pharmaceutical composition comprising a compound of formula (I) or a pharmaceutically acceptable salt thereof and fulvestrant

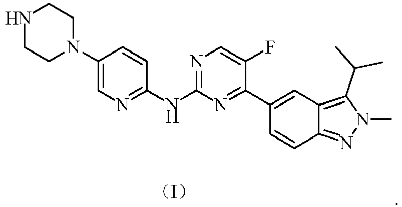

In another aspect, the present disclosure provides a combined pharmaceutical composition for use in treating or preventing breast cancer, comprising a compound of formula (I) or a pharmaceutically acceptable salt thereof and fulvestrant.

In some embodiments of the present disclosure, the combined pharmaceutical composition is packaged in a kit, and the kit further comprises an instruction for using the compound of formula (I) or the pharmaceutically acceptable salt thereof in combination with fulvestrant to treat or prevent breast cancer.

In some embodiments of the present disclosure, the combined pharmaceutical composition comprises: a pharmaceutical composition of the compound of formula (I) or the pharmaceutically acceptable salt thereof, and a pharmaceutical composition of fulvestrant.

In some embodiments of the present disclosure, the combined pharmaceutical composition is packaged in a kit, and the kit further comprises an instruction for using the pharmaceutical composition of the compound of formula (I) or the pharmaceutically acceptable salt thereof in combination with the pharmaceutical composition of fulvestrant to treat or prevent breast cancer.

In some embodiments of the present disclosure, the pharmaceutically acceptable salt of the compound of formula (I) in the combined pharmaceutical composition is a maleate, such as a monomaleate of the compound of formula (I).

In some embodiments of the present disclosure, the combined pharmaceutical composition comprises 20-240 mg, 40-180 mg, 60-180 mg, 80-180 mg, 100-180 mg, 120-180 mg, 150-180 mg, or 150-240 mg of the compound of formula (I) or the pharmaceutically acceptable salt thereof based on the compound of formula (I), or a pharmaceutical composition thereof.

In some embodiments of the present disclosure, the combined pharmaceutical composition comprises 20-240 mg, 40-180 mg, 60-180 mg, 80-180 mg, 100-180 mg, 120-180 mg, or 150-180 mg of the compound of formula (I) or the pharmaceutically acceptable salt thereof based on the compound of formula (I), or a pharmaceutical composition thereof.

In some embodiments of the present disclosure, the combined pharmaceutical composition comprises 150-240 mg of the compound of formula (I) or the pharmaceutically acceptable salt thereof based on the compound of formula (I), or a pharmaceutical composition thereof.

In some embodiments of the present disclosure, the combined pharmaceutical composition comprises 20 mg, 40 mg, 60 mg, 80 mg, 100 mg, 120 mg, 150 mg, 180 mg, or 240 mg of the compound of formula (I) or the pharmaceutically acceptable salt thereof based on the compound of formula (I), or a pharmaceutical composition thereof.

In some embodiments of the present disclosure, the combined pharmaceutical composition comprises 60 mg, 120 mg, or 180 mg of the compound of formula (I) or the pharmaceutically acceptable salt thereof based on the compound of formula (I), or a pharmaceutical composition thereof.

In some embodiments of the present disclosure, the combined pharmaceutical composition comprises 150 mg or 180 mg of the compound of formula (I) or the pharmaceutically acceptable salt thereof based on the compound of formula (I), or a pharmaceutical composition thereof.

In some embodiments of the present disclosure, the combined pharmaceutical composition comprises 180 mg of the compound of formula (I) or the pharmaceutically acceptable salt thereof based on the compound of formula (I), or a pharmaceutical composition thereof.

In some embodiments of the present disclosure, in the combined pharmaceutical composition, the pharmaceutical composition of the compound of formula (I) or the pharmaceutically acceptable salt thereof is in a single dose or in a multi-dose form. In some embodiments of the present disclosure, in the combined pharmaceutical composition, the pharmaceutical composition of the compound of formula (I) or the pharmaceutically acceptable salt thereof is in a multi-dose form.

In some embodiments of the present disclosure, in the combined pharmaceutical composition, the content of the compound of formula (I) or the pharmaceutically acceptable salt thereof is a daily dose.

In some embodiments of the present disclosure, in the combined pharmaceutical composition, the content of the compound of formula (I) or the pharmaceutically acceptable salt thereof is a once-daily dose.

In some embodiments of the present disclosure, in the combined pharmaceutical composition, the content of the compound of formula (I) or the pharmaceutically acceptable salt thereof is a once-daily dose, with each dose being a single dose or multiple doses, typically multiple doses.

In some embodiments of the present disclosure, in the combined pharmaceutical composition, the combined pharmaceutical composition comprises the pharmaceutical composition of the compound of formula (I) or the pharmaceutically acceptable salt thereof in a single dose of 5 mg, 50 mg, or 60 mg, based on the compound of formula (I). Alternatively, the combined pharmaceutical composition is in the form of a single administration, and the combined pharmaceutical composition comprises 5 mg, 50 mg, or 60 mg of the compound of formula (I) or the pharmaceutically acceptable salt thereof based on the compound of formula (I), or a pharmaceutical composition thereof.

In some embodiments of the present disclosure, in the combined pharmaceutical composition, the combined pharmaceutical composition comprises the pharmaceutical composition of the compound of formula (I) or the pharmaceutically acceptable salt thereof in a single dose of 60 mg, based on the compound of formula (I). Alternatively, the combined pharmaceutical composition is in the form of a single administration, and the combined pharmaceutical composition comprises 60 mg of the compound of formula (I) or the pharmaceutically acceptable salt thereof based on the compound of formula (I), or a pharmaceutical composition thereof.

In some embodiments of the present disclosure, in the combined pharmaceutical composition, the pharmaceutical composition of the compound of formula (I) or the pharmaceutically acceptable salt thereof comprised in the combined pharmaceutical composition is packaged in a kit, and the kit further comprises an instruction for use of the compound of formula (I) or the pharmaceutically acceptable salt thereof in treating or preventing breast cancer.

In some embodiments of the present disclosure, the combined pharmaceutical composition comprises 125-1000 mg, 125-750 mg, 250-750 mg, or 250-500 mg of fulvestrant, or a pharmaceutical composition thereof.

In some embodiments of the present disclosure, the combined pharmaceutical composition comprises 125 mg, 250 mg, 500 mg, 750 mg, or 1000 mg of fulvestrant, or a pharmaceutical composition thereof.

In some embodiments of the present disclosure, the combined pharmaceutical composition comprises 250 mg or 500 mg of fulvestrant, or a pharmaceutical composition thereof.

In some embodiments of the present disclosure, the combined pharmaceutical composition comprises 500 mg of fulvestrant, or a pharmaceutical composition thereof.

In some embodiments of the present disclosure, in the combined pharmaceutical composition, the pharmaceutical composition of fulvestrant is in a single dose or in a multi-dose form.

In some embodiments of the present disclosure, in the combined pharmaceutical composition, the pharmaceutical composition of fulvestrant is in a multi-dose form.

In some embodiments of the present disclosure, in the combined pharmaceutical composition, a content of fulvestrant is a dose per treatment cycle.

In some embodiments of the present disclosure, in the combined pharmaceutical composition, the content of fulvestrant is a dose to be administered once per treatment cycle.

In some embodiments of the present disclosure, in the combined pharmaceutical composition, the combined pharmaceutical composition comprises a pharmaceutical composition of fulvestrant in a single dose of 250 mg. Alternatively, the combined pharmaceutical composition is in the form of a single administration, and the combined pharmaceutical composition comprises 250 mg of fulvestrant or a pharmaceutical composition thereof. In some embodiments of the present disclosure, in the combined pharmaceutical composition, the pharmaceutical composition of fulvestrant comprised in the combined pharmaceutical composition is packaged in a kit, and the kit further comprises an instruction for use of fulvestrant in treating or preventing breast cancer.

In some embodiments of the present disclosure, in the combined pharmaceutical composition, the pharmaceutical composition of fulvestrant comprised in the combined pharmaceutical composition is packaged in a kit, the kit further comprises an instruction for use of fulvestrant in treating or preventing breast cancer, and the instruction may be the instruction of a commercially available fulvestrant injection kit. In some embodiments of the present disclosure, the instruction is the instruction of the fulvestrant injection produced by Chia Tai Tianqing Pharmaceutical Group Co., Ltd. (e.g., Qingkeyi^{®}).

In some embodiments of the present disclosure, the combined pharmaceutical composition comprises 20-240 mg of the compound of formula (I) or the pharmaceutically acceptable salt thereof based on the compound of formula (I), and 125-1000 mg of fulvestrant.

In some embodiments of the present disclosure, the combined pharmaceutical composition comprises 120-180 mg of the compound of formula (I) or the pharmaceutically acceptable salt thereof based on the compound of formula (I), and 250-500 mg of fulvestrant.

In some embodiments of the present disclosure, the combined pharmaceutical composition comprises 20 mg, 40 mg, 60 mg, 80 mg, 100 mg, 120 mg, 150 mg, 180 mg, or 240 mg of a daily dose of the compound of formula (I) or the pharmaceutically acceptable salt thereof based on the compound of formula (I), and 250 mg or 500 mg of a dose per treatment cycle of fulvestrant.

In some embodiments of the present disclosure, the combined pharmaceutical composition comprises 60 mg, 120 mg, 180 mg, or 240 mg of a daily dose of the compound of formula (I) or the pharmaceutically acceptable salt thereof based on the compound of formula (I), and 250 mg or 500 mg of a dose per treatment cycle of fulvestrant. In some embodiments of the present disclosure, the combined pharmaceutical composition comprises 150 mg or 180 mg of a daily dose of the compound of formula (I) or the pharmaceutically acceptable salt thereof based on the compound of formula (I), and 500 mg of a dose per treatment cycle of fulvestrant.

In some embodiments of the present disclosure, the combined pharmaceutical composition comprises 150 mg or 180 mg of a daily dose of the compound of formula (I) or the pharmaceutically acceptable salt thereof based on the compound of formula (I), and 1000 mg of a dose at a first treatment cycle of fulvestrant.

In some embodiments of the present disclosure, the combined pharmaceutical composition comprises 150 mg or 180 mg of a daily dose of the compound of formula (I) or the pharmaceutically acceptable salt thereof based on the compound of formula (I), and 1000 mg of a dose at a first treatment cycle of fulvestrant and subsequent 500 mg of a dose per treatment cycle of fulvestrant.

In some embodiments of the present disclosure, the combined pharmaceutical composition comprises 180 mg of a daily dose of the compound of formula (I) or the pharmaceutically acceptable salt thereof based on the compound of formula (I), and 500 mg of a dose per treatment cycle of fulvestrant.

In some embodiments of the present disclosure, the combined pharmaceutical composition comprises 180 mg of a daily dose of the compound of formula (I) or the pharmaceutically acceptable salt thereof based on the compound of formula (I), and 1000 mg of a dose at a first treatment cycle of fulvestrant and subsequent 500 mg of a dose per treatment cycle of fulvestrant.

In some embodiments of the present disclosure, every 28 days are counted as one treatment cycle.

In some embodiments of the present disclosure, the combined pharmaceutical composition comprises a pharmaceutical composition of the compound of formula (I) or the pharmaceutically acceptable salt thereof in a single dose of 50 mg based on the compound of formula (I), and a pharmaceutical composition of fulvestrant in a single dose of 250 mg based on fulvestrant. Alternatively, the combined pharmaceutical composition is in the form of a single administration, and the combined pharmaceutical composition comprises 50 mg of the compound of formula (I) or the pharmaceutically acceptable salt thereof based on the compound of formula (I), or a pharmaceutical composition thereof, and 250 mg of fulvestrant based on fulvestrant or a pharmaceutical composition thereof.

In some embodiments of the present disclosure, the combined pharmaceutical composition comprises a pharmaceutical composition of the compound of formula (I) or the pharmaceutically acceptable salt thereof in a single dose of 60 mg based on the compound of formula (I), and comprises a pharmaceutical composition of fulvestrant in a single dose of 250 mg based on fulvestrant. Alternatively, the combined pharmaceutical composition is in the form of a single administration, and the combined pharmaceutical composition comprises 60 mg of the compound of formula (I) or the pharmaceutically acceptable salt thereof based on the compound of formula (I), or a pharmaceutical composition thereof, and 250 mg of fulvestrant based on fulvestrant or a pharmaceutical composition thereof.

In some embodiments of the present disclosure, the combined pharmaceutical composition is a formulation suitable for administration within a single treatment cycle (e.g., a treatment cycle of 28 days). The formulation comprises a pharmaceutical composition comprising the compound of formula (I) or the pharmaceutically acceptable salt thereof in a total dose of 1680-5040 mg (e.g., 3360 mg, 4200 mg, or 5040 mg) based on the compound of formula (I), and a pharmaceutical composition comprising fulvestrant in a total dose of 500-1000 mg based on fulvestrant.

In some embodiments of the present disclosure, the combined pharmaceutical composition is a formulation suitable for administration within a single treatment cycle (e.g., a treatment cycle of 28 days). The formulation comprises a pharmaceutical composition comprising the compound of formula (I) or the pharmaceutically acceptable salt thereof in a total dose of 5040 mg based on the compound of formula (I), and a pharmaceutical composition comprising fulvestrant in a total dose of 500-1000 mg based on fulvestrant.

In some embodiments of the present disclosure, the combined pharmaceutical composition is a formulation suitable for administration within a first treatment cycle (e.g., a treatment cycle of 28 days). The formulation comprises a pharmaceutical composition comprising the compound of formula (I) or the pharmaceutically acceptable salt thereof in a total dose of 5040 mg based on the compound of formula (I), and a pharmaceutical composition comprising fulvestrant in a total dose of 1000 mg based on fulvestrant.

In some embodiments of the present disclosure, the combined pharmaceutical composition is a formulation suitable for administration within a single treatment cycle (e.g., a treatment cycle of 28 days) after a second treatment cycle. The formulation comprises a pharmaceutical composition comprising the compound of formula (I) or the pharmaceutically acceptable salt thereof in a total dose of 5040 mg based on the compound of formula (I), and a pharmaceutical composition comprising fulvestrant in a total dose of 500 mg based on fulvestrant.

In some embodiments of the present disclosure, the combined pharmaceutical composition is a formulation suitable for administration within a single treatment cycle (e.g., a treatment cycle of 28 days), and the formulation comprises a pharmaceutical composition comprising the compound of formula (I) or the pharmaceutically acceptable salt thereof and a pharmaceutical composition comprising fulvestrant in a total dose ratio of (1-30): 1, e.g., (1-25):1, (1.5-25):1, (1.5-20):1, (1.5-15):1, or (1.5-12):1, or any ratio within the range described above, wherein the dose of the pharmaceutical composition comprising the compound of formula (I) or the pharmaceutically acceptable salt thereof is based on the compound of formula (I) and the dose of the pharmaceutical composition comprising fulvestrant is based on fulvestrant.

In some embodiments of the present disclosure, in the combined pharmaceutical composition, the compound of formula (I) or the pharmaceutically acceptable salt thereof and fulvestrant may be each in a form of a pharmaceutical composition or be in a form of a pharmaceutical composition together.

In another aspect, the present disclosure also provides a kit comprising (a) a first pharmaceutical composition comprising the compound of formula (I) or the pharmaceutically acceptable salt thereof described herein; and (b) a second pharmaceutical composition comprising fulvestrant.

In another aspect, the present disclosure also provides a kit of a pharmaceutical composition for use in treating or preventing breast cancer, comprising (a) a first pharmaceutical composition comprising the compound of formula (I) or the pharmaceutically acceptable salt thereof described herein; and (b) a second pharmaceutical composition comprising fulvestrant. Alternatively, the present disclosure also provides a combined pharmaceutical composition for use in treating or preventing breast cancer, comprising a pharmaceutical composition of the compound of formula (I) or the pharmaceutically acceptable salt thereof, and a pharmaceutical composition of fulvestrant.

In some embodiments of the present disclosure, in the combined pharmaceutical composition, the compound of formula (I) or the pharmaceutically acceptable salt thereof is prepared to comprise 5 mg, 50 mg, or 60 mg of the compound of formula (I) or the pharmaceutically acceptable salt thereof based on the compound of formula (I) in a unit formulation, and the fulvestrant is prepared to comprise 250 mg of fulvestrant in a unit formulation.

In some embodiments of the present disclosure, in the combined pharmaceutical composition, the compound of formula (I) or the pharmaceutically acceptable salt thereof is prepared to comprise 50 mg or 60 mg of the compound of formula (I) or the pharmaceutically acceptable salt thereof based on the compound of formula (I) in a unit formulation, and the fulvestrant is prepared to comprise 250 mg of fulvestrant in a unit formulation.

In another aspect, the present disclosure also provides a method of treating or preventing breast cancer, comprising administering to an individual in need thereof a therapeutically effective amount of the compound of formula (I) or the pharmaceutically acceptable salt thereof and fulvestrant, for example, administering to an individual in need thereof a therapeutically effective amount of the combined pharmaceutical composition described above of the present disclosure.

In another aspect, the present disclosure also provides a combination therapy for treating an individual with breast cancer, comprising administering to the individual a therapeutically effective amount of the compound of formula (I) or the pharmaceutically acceptable salt thereof alone and a therapeutically effective amount of fulvestrant alone.

In another aspect, the present disclosure also provides use of the compound of formula (I) or the pharmaceutically acceptable salt thereof in combination with fulvestrant for preparing a medicament for use in treating or preventing breast cancer, for example, use of the combined pharmaceutical composition described above in the present disclosure for preparing a medicament for use in treating or preventing breast cancer. In some embodiments of the present disclosure, the combined pharmaceutical composition is any of the combined pharmaceutical compositions described above in the present disclosure.

In another aspect, the present disclosure also provides use of the compound of formula (I) or the pharmaceutically acceptable salt thereof in combination with fulvestrant for treating or preventing breast cancer, for example, use of the combined pharmaceutical composition described above in the present disclosure for treating or preventing breast cancer.

In some embodiments of the present disclosure, in the kit, the method, the combination therapy, or the use, each of the definitions of the compound of formula (I) or the pharmaceutically acceptable salt thereof and fulvestrant is the same as the definitions for the compound of formula (I) or the pharmaceutically acceptable salt thereof and fulvestrant in the combined pharmaceutical composition described above, e.g., in content, dose, presentation form, packaging form or the like.

In some embodiments of the present disclosure, in the method, the combination therapy, or the use, the compound of formula (I) or the pharmaceutically acceptable salt thereof and fulvestrant are each in a form of a pharmaceutical composition, and can be administered simultaneously, separately, concurrently, sequentially, or at intervals.

In some embodiments of the present disclosure, in the method, the combination therapy, or the use, the compound of formula (I) or the pharmaceutically acceptable salt thereof and fulvestrant each have the same or different treatment cycles. In some specific embodiments of the present disclosure, in the method, the combination therapy, or the use, the compound of formula (I) or the pharmaceutically acceptable salt thereof and fulvestrant have the same treatment cycle, e.g., a 1-week, 2-week, 3-week, or 4-week treatment cycle.

In some embodiments of the present disclosure, in the method, the combination therapy, or the use, a content of the compound of formula (I) or the pharmaceutically acceptable salt thereof in the combined pharmaceutical composition is a daily dose, which is administered by administering the compound of formula (I) or the pharmaceutically acceptable salt thereof once daily.

In some embodiments of the present disclosure, in the method, the combination therapy, or the use, a content of the compound of formula (I) or the pharmaceutically acceptable salt thereof in the combined pharmaceutical composition is a daily dose, wherein the compound of formula (I) or the pharmaceutically acceptable salt thereof is administered in a single dose or in a multi-dose form, typically in a multi-dose form; further, the compound of formula (I) or the pharmaceutically acceptable salt thereof is administered once daily.

In some embodiments of the present disclosure, in the method, the combination therapy, or the use, the compound of formula (I) or the pharmaceutically acceptable salt thereof is administered in a daily dose of 60 mg, or in a daily dose of 120 mg, or in a daily dose of 180 mg, or in a daily dose of 240 mg.

In some embodiments of the present disclosure, in the method, the combination therapy, or the use, the compound of formula (I) or the pharmaceutically acceptable salt thereof is administered in a daily dose of 150 mg, or in a daily dose of 180 mg.

In some embodiments of the present disclosure, in the method, the combination therapy, or the use, the compound of formula (I) or the pharmaceutically acceptable salt thereof is administered in a daily dose of 180 mg.

In some embodiments of the present disclosure, in the method, the combination therapy, or the use, the compound of formula (I) or the pharmaceutically acceptable salt thereof in the combined pharmaceutical composition is administered in a multi-dose form consisting of a single dose of 50 mg of the pharmaceutical composition of the compound of formula (I) or the pharmaceutically acceptable salt thereof. In some embodiments of the present disclosure, in the method, the combination therapy, or the use, the compound of formula (I) or the pharmaceutically acceptable salt thereof is administered in a consecutive daily administration.

In some embodiments of the present disclosure, in the method, the combination therapy, or the use, the compound of formula (I) or the pharmaceutically acceptable salt thereof in the combined pharmaceutical composition is administered in a multi-dose form consisting of a single dose of 60 mg of the pharmaceutical composition of the compound of formula (I) or the pharmaceutically acceptable salt thereof. In some embodiments of the present disclosure, in the method, the combination therapy, or the use, the compound of formula (I) or the pharmaceutically acceptable salt thereof is administered in a consecutive daily administration.

In some embodiments of the present disclosure, in the method, the combination therapy, or the use, a content of the compound of formula (I) or the pharmaceutically acceptable salt thereof in the combined pharmaceutical composition is a dose per treatment cycle, which is administered by administering the compound of formula (I) or the pharmaceutically acceptable salt thereof daily. The compound of formula (I) or the pharmaceutically acceptable salt thereof is packaged in a single aliquot or in multiple aliquots (e.g., 2, 4, 7, 14, 28 or more aliquots). In some embodiments of the present disclosure, in the method, the combination therapy, or the use, a content of fulvestrant in the combined pharmaceutical composition is a dose per treatment cycle, which is administered by administering fulvestrant on day 1 and day 15 in the first treatment cycle, and on day 1 in each subsequent treatment cycle. Fulvestrant is packaged in a single aliquot or in multiple aliquots (e.g., 2, 4 or more aliquots). In some embodiments of the present disclosure, fulvestrant is administered on day 1 and day 15 in the first treatment cycle, each dose being the same, and on day 1 in each subsequent treatment cycle, the dose on day 1 in each treatment cycle being the same as the dose on day 1 in the first treatment cycle. In some embodiments of the present disclosure, the dose is the same for each administration during the treatment cycle of fulvestrant.

In some embodiments of the present disclosure, in the method, the combination therapy, or the use, a content of fulvestrant in the combined pharmaceutical composition is a dose per treatment cycle, wherein fulvestrant is administered in a single dose or in a multi-dose form. In some embodiments of the present disclosure, fulvestrant is administered in a multi-dose form.

In some embodiments of the present disclosure, in the method, the combination therapy, or the use, a content of fulvestrant in the combined pharmaceutical composition is a dose per treatment cycle, wherein fulvestrant is administered in a multi-dose form consisting of a single dose of 250 mg of the pharmaceutical composition of fulvestrant.

In some embodiments of the present disclosure, in the method, the combination therapy, or the use, 28 days are counted as one treatment cycle, fulvestrant is administered on day 1 and day 15 in the first cycle and on day 1 in each subsequent treatment cycle, and the compound of formula (I) or the pharmaceutically acceptable salt thereof is administered daily on days 1-28 in each treatment cycle.

In some specific embodiments of the present disclosure, in the method, the combination therapy, or the use, 28 days are counted as one treatment cycle, fulvestrant is administered once on each of day 1 and day 15 in the first cycle and once on day 1 in each subsequent treatment cycle, and the compound of formula (I) or the pharmaceutically acceptable salt thereof is administered once daily on days 1-28 in each treatment cycle.

In some embodiments of the present disclosure, in the method, the combination therapy, or the use, 28 days are counted as one treatment cycle, the administration is performed once daily for 28 days, and a total dose of the pharmaceutical composition comprising the compound of formula (I) or the pharmaceutically acceptable salt thereof administered per treatment cycle is 1680-5040 mg. In some embodiments, a total dose of the pharmaceutical composition comprising the compound of formula (I) or the pharmaceutically acceptable salt thereof is selected from the group consisting of 1680 mg, 3360 mg, 4200 mg, and 5040 mg or a range formed by any two of the aforementioned values. In some embodiments, the total dose of the pharmaceutical composition comprising the compound of formula (I) or the pharmaceutically acceptable salt thereof is preferably 4200 mg or 5040 mg. In some embodiments, the total dose of the pharmaceutical composition comprising the compound of formula (I) or the pharmaceutically acceptable salt thereof is preferably 5040 mg.

In some embodiments of the present disclosure, in the method, the combination therapy, or the use, 28 days are counted as one treatment cycle, and 500 mg of fulvestrant is administered once on each of day 1 and day 15 in the first treatment cycle and once on day 1 in each subsequent treatment cycle.

In some embodiments of the present disclosure, in the method, the combination therapy, or the use, 28 days are counted as one treatment cycle, the pharmaceutical composition comprising the compound of formula (I) or the pharmaceutically acceptable salt thereof is administered once daily for 28 days, and a total dose of the pharmaceutical composition comprising the compound of formula (I) or the pharmaceutically acceptable salt thereof administered per treatment cycle is 5040 mg; 500 mg of fulvestrant is administered once on each of day 1 and day 15 in the first treatment cycle and once on day 1 in each subsequent treatment cycle.

In embodiments of the present disclosure, the treatment cycle described above is repeated as long as the disease is still under control and the administration regimen is clinically tolerable.

In some embodiments of the present disclosure, the fulvestrant is prepared as a single dose or multiple doses suitable for administering 250-750 mg or 250-500 mg of fulvestrant to a patient per treatment cycle; the pharmaceutical composition comprising the compound of formula (I) or the pharmaceutically acceptable salt thereof is prepared as a single dose or multiple doses suitable for continuously administering daily 60 mg, 120 mg, 180 mg, and/or 240 mg based on the compound of formula (I) to a patient.

In some embodiments of the present disclosure, the fulvestrant is prepared as a single dose or multiple doses suitable for administering 250-750 mg or 250-500 mg of fulvestrant to a patient per treatment cycle; the pharmaceutical composition comprising the compound of formula (I) or the pharmaceutically acceptable salt thereof is prepared as a single dose or multiple doses suitable for continuously administering daily 150 mg and/or 180 mg based on the compound of formula (I) to a patient.

In some embodiments of the present disclosure, in the method, the combination therapy, or the use, 125 mg, 250 mg, 500 mg, 750 mg, or 1000 mg of fulvestrant is administered to the patient per treatment cycle; or 250 mg or 500 mg of fulvestrant is administered to the patient per treatment cycle.

In embodiments of the present disclosure, in the method, the combination therapy, or the use, fulvestrant is administered on day 1 and day 15 in 28 days of the first treatment cycle, each counted as one treatment cycle administration.

In some embodiments of the present disclosure, the compound of formula (I) or the pharmaceutically acceptable salt thereof is prepared as a single dose or multiple doses suitable for continuously administering daily 60 mg, 120 mg, 180 mg, and/or 240 mg of the compound of formula (I) or the pharmaceutically acceptable salt thereof to a patient; or the compound of formula (I) or the pharmaceutically acceptable salt thereof is prepared as a single dose or multiple doses suitable for continuously administering daily 120 mg or 180 mg based on the compound of formula (I) or the pharmaceutically acceptable salt thereof to a patient.

In some embodiments of the present disclosure, the compound of formula (I) or the pharmaceutically acceptable salt thereof is prepared as a single dose or multiple doses suitable for continuously administering daily 60 mg, 120 mg, 180 mg, and/or 240 mg of the compound of formula (I) or the pharmaceutically acceptable salt thereof to a patient; or the compound of formula (I) or the pharmaceutically acceptable salt thereof is prepared as a single dose or multiple doses suitable for continuously administering daily 150 mg or 180 mg based on the compound of formula (I) or the pharmaceutically acceptable salt thereof to a patient.

In some embodiments of the present disclosure, the breast cancer is selected from the group consisting of HR-positive and HER2-negative breast cancer.

In some embodiments of the present disclosure, the breast cancer is selected from the group consisting of locally advanced and/or metastatic breast cancer.

In some embodiments of the present disclosure, the breast cancer is selected from the group consisting of HR-positive and HER2-negative locally advanced and/or metastatic breast cancer.

In some embodiments of the present disclosure, the breast cancer is selected from the group consisting of HR-positive and HER2-negative locally advanced and/or metastatic breast cancer unable to receive radical surgery or radiotherapy.

In some embodiments of the present disclosure, the breast cancer is selected from the group consisting of postmenopausal or premenopausal/perimenopausal breast cancer.

In some embodiments of the present disclosure, the breast cancer is selected from the group consisting of postmenopausal or premenopausal/perimenopausal HR-positive and HER2-negative locally advanced and/or metastatic breast cancer.

In some embodiments of the present disclosure, the breast cancer is selected from the group consisting of breast cancer previously received bilateral ovariectomy.

In some embodiments of the present disclosure, the breast cancer is selected from the group consisting of postmenopausal or premenopausal/perimenopausal breast cancer previously received bilateral ovariectomy.

In some embodiments of the present disclosure, the breast cancer is selected from the group consisting of postmenopausal or premenopausal/perimenopausal HR-positive and HER2-negative locally advanced and/or metastatic breast cancer previously received bilateral ovariectomy.

In some embodiments of the present disclosure, the breast cancer is selected from the group consisting of postmenopausal or premenopausal/perimenopausal HR-positive and HER2-negative locally advanced and/or metastatic breast cancer unable to receive radical surgery or radiotherapy.

In some embodiments of the present disclosure, the HR-positive includes estrogen receptor (ER)-positive and/or progesterone receptor (PR)-positive, and is defined as: the proportion of positive staining tumor cells in all tumor cells is more than or equal to 1%.

In some embodiments of the present disclosure, HER2-negative is defined as: HER2 is 0/1+ as detected by immunohistochemical staining (IHC); if HER2 is 2+ as detected, fluorescence in situ hybridization (FISH) should be performed to confirm that the result is negative, or only FISH assay is performed and the result is negative.

In some embodiments of the present disclosure, the breast cancer is selected from the group consisting of HR-positive and HER2-negative locally advanced and/or metastatic breast cancer previously received chemotherapy no more than first-line.

In some embodiments of the present disclosure, the breast cancer is selected from the group consisting of HR-positive and HER2-negative breast cancer that relapse or progress during an adjuvant endocrine therapy or within 1 year after the completion of an adjuvant endocrine therapy and have not received endocrine therapy subsequently.

In some embodiments of the present disclosure, the breast cancer is selected from the group consisting of HR-positive and HER2-negative breast cancer that relapse or progress over 1 year after the completion of an adjuvant endocrine therapy and subsequently progress again after receiving an advanced endocrine therapy; the advanced endocrine therapy is no more than first-line.

In some embodiments of the present disclosure, the breast cancer is selected from the group consisting of breast cancer with disease progression after receiving an advanced endocrine therapy for a primary or metastatic disease; the advanced endocrine therapy is no more than first-line.

In some embodiments of the present disclosure, the breast cancer is selected from the group consisting of HR-positive and HER2-negative locally advanced and/or metastatic breast cancer that has not previously received any systemic anti-tumor therapy for local lesion recurrence or metastatic diseases.

In some embodiments of the present disclosure, the endocrine therapy is tamoxifen, toremifene, fulvestrant, letrozole, anastrozole, exemestane, goserelin, and leuprorelin therapy.

In some embodiments of the present disclosure, the endocrine therapy is tamoxifen, letrozole, exemestane, and goserelin therapy.

In some embodiments of the present disclosure, the endocrine therapy is tamoxifen and goserelin therapy.

The active ingredients in the pharmaceutical combination disclosed herein can be formulated independently, or some or all of the active ingredients are co-formulated with a pharmaceutically acceptable carrier and/or excipient. The pharmaceutical combination disclosed herein may further comprise an additional therapeutic agent. In some embodiments of the present disclosure, the additional therapeutic agent may be a therapeutic agent known in the art for cancer, preferably a therapeutic agent for breast cancer.

In some embodiments of the present disclosure, the cycle is 28 days.

The amount of the compound of formula (I) or the pharmaceutically acceptable salt thereof administered can be determined according to the severity of the disease, the response of the disease, any treatment-related toxicity, and the age and health of a patient.

The compound of formula (I) or the pharmaceutically acceptable salt thereof can be administered via multiple routes of administration including, but not limited to, oral, parenteral, intraperitoneal, intravenous, intra-arterial, transdermal, sublingual, intramuscular, rectal, transbuccal, intranasal, inhalational, vaginal, intraocular, topical, subcutaneous, intra-adipose, intra-articular, intraperitoneal, and intrathecal administrations. In a specific embodiment, the compound of formula (I) or the pharmaceutically acceptable salt thereof is administered orally. In some embodiments of the present disclosure, the compound of formula (I) or the pharmaceutically acceptable salt thereof is administered in a consecutive daily oral administration.

The compound of formula (I) or the pharmaceutically acceptable salt thereof can be administered once or multiple times daily. In some embodiments of the present disclosure, the compound of formula (I) or the pharmaceutically acceptable salt thereof is administered once daily. The compound of formula (I) or the pharmaceutically acceptable salt thereof may also be administered in a single dose or in a multi-dose form. In one embodiment, the compound of formula (I) or the pharmaceutically acceptable salt thereof is administered in multiple doses once daily.

In some embodiments of the present disclosure, the compound of formula (I) or the pharmaceutically acceptable salt thereof is administered in a form of a multi-dose oral solid formulation once daily. In one embodiment, the compound of formula (I) or the pharmaceutically acceptable salt thereof is administered in multiple doses once daily.

The dosage regimen can be determined comprehensively depending on the activity and toxicity of the medicament, tolerance of a patient, etc.

### Compound of formula (I) or pharmaceutically acceptable salt thereof

The compound of formula (I) of the present disclosure may be administered in its free base form, or in the form of its pharmaceutically acceptable salt, hydrate or prodrug that converts *in vivo* into the form of the compound of formula (I). For example, within the scope of the present disclosure, the pharmaceutically acceptable salt of the compound of formula (I) can be generated from various organic and inorganic acids according to methods well known in the art.

With respect to the pharmaceutically acceptable salt of the compound of formula (I) described herein, a molar ratio of the compound of formula (I) to an acid radical involved in the formation of the pharmaceutically acceptable salt may be 1:1.

The pharmaceutically acceptable salt of the compound of formula (I) may be a maleate of the compound of formula (I) (such as a monomaleate of the compound of formula (I)).

The dosage of the compound of formula (I) or the pharmaceutically acceptable salt thereof referred to in the present disclosure is based on the amount of the compound of formula (I), unless otherwise stated.

In some embodiments of the present disclosure, the pharmaceutically acceptable salt of the compound of formula (I) is present in a form of a salt of the compound of formula (I).

The compound of formula (I) or the pharmaceutically acceptable salt thereof used in the present disclosure may be prepared by methods known in the art, for example, by reference to WO2016141881.

### Pharmaceutical composition of compound of formula (I) or pharmaceutically acceptable salt thereof

In some embodiments of the present disclosure, a single dose of the pharmaceutical composition of the compound of formula (I) or the pharmaceutically acceptable salt thereof is 50 mg or 60 mg, based on the compound of formula (I). Alternatively, the pharmaceutical composition of the compound of formula (I) or the pharmaceutically acceptable salt thereof is prepared to comprise 50 mg or 60 mg of the compound of formula (I) or the pharmaceutically acceptable salt thereof based on the compound of formula (I) in a unit formulation.

In some embodiments of the present disclosure, a single dose of the pharmaceutical composition of the compound of formula (I) or the pharmaceutically acceptable salt thereof is 60 mg based on the compound of formula (I). Alternatively, the pharmaceutical composition of the compound of formula (I) or the pharmaceutically acceptable salt thereof is prepared to comprise 60 mg of the compound of formula (I) or the pharmaceutically acceptable salt thereof based on the compound of formula (I) in a unit formulation.

The dosage regimen can be determined comprehensively depending on the activity and toxicity of the medicament, tolerance of a patient, etc.

In some embodiments of the present disclosure, the pharmaceutical composition comprising the compound of formula (I) or the pharmaceutically acceptable salt thereof further comprises a pharmaceutically acceptable excipient. The pharmaceutically acceptable excipient includes fillers, absorbents, wetting agents, binders, disintegrants, lubricants, and the like. In some embodiments of the present disclosure, the pharmaceutical composition includes, but is not limited to, a formulation suitable for oral, parenteral and topical administration. In some embodiments, the pharmaceutical composition is a formulation suitable for oral administration. In some embodiments, the pharmaceutical composition is a solid formulation suitable for oral administration. In some embodiments, the pharmaceutical composition includes, but is not limited to, tablets and capsules.

In some embodiments of the present disclosure, the pharmaceutical composition is a solid pharmaceutical combination.

In some embodiments of the present disclosure, the pharmaceutical composition of the compound of formula (I) or the pharmaceutically acceptable salt thereof is a solid pharmaceutical composition comprising the compound of formula (I) or the pharmaceutically acceptable salt thereof.

In some embodiments of the present disclosure, the pharmaceutical composition of the compound of formula (I) or the pharmaceutically acceptable salt thereof is a capsule comprising the compound of formula (I) or the pharmaceutically acceptable salt thereof.

The pharmaceutical composition disclosed herein can be manufactured using methods well known in the art, such as conventional mixing, dissolving, granulating, dragee-making, levigating, emulsifying, and lyophilizing.

A solid oral composition can be prepared by conventional mixing, filling or tableting. For example, it can be obtained by the following method: mixing the active compounds with solid excipients, optionally grinding the resulting mixture, adding additional suitable excipients if desired, and processing the mixture into granules to get the core parts of tablets or dragees. Suitable excipients include, but are not limited to: binders, diluents, disintegrants, lubricants, glidants, sweeteners or flavoring agents and the like.

### Fulvestrant

As used herein, the chemical name of fulvestrant is 7-α-[9-(4,4,5,5,5-pentafluoropentylsulfinyl)nonyl]estra-1,3,5-(10)-triene-3,17-β-diol, which has a structure of a compound of formula (II) below:

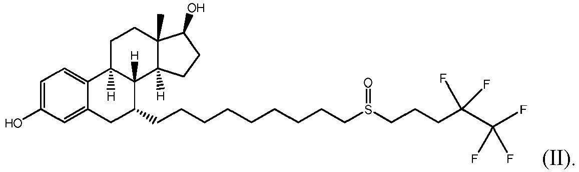

### Pharmaceutical composition of fulvestrant

In some embodiments of the present disclosure, the pharmaceutical composition of fulvestrant further comprises a pharmaceutically acceptable excipient. Preferably, the pharmaceutically acceptable excipient includes fillers, absorbents, wetting agents, binders, disintegrants, lubricants, water for injection, and the like.

In some embodiments of the present disclosure, the pharmaceutical composition is an injection.

In some embodiments of the present disclosure, the pharmaceutical composition is a water-soluble injection, including but not limited to a water-soluble formulation without lyophilization or a water-soluble formulation reconstituted from a lyophilized powder.

In some embodiments of the present disclosure, a single dose of the pharmaceutical composition of the compound of formula (II) or a pharmaceutically acceptable salt thereof is 250 mg.

In some embodiments of the present disclosure, an amount of fulvestrant in the pharmaceutical composition of fulvestrant is 250 mg or 500 mg.

Fulvestrant injections used in the present disclosure may be commercially available.

### Administration

The content below is not intended to limit the administration of the combined pharmaceutical composition disclosed herein.

The active ingredients in the combined pharmaceutical composition disclosed herein can be administered independently, or some or all of the active ingredients are co-administered in various proper routes, including, but not limited to, oral administration or parenteral administration (by intravenous, intramuscular, topical, or subcutaneous routes). In some embodiments of the present disclosure, the active ingredients in the combined pharmaceutical composition disclosed herein can be administered independently, or some or all of the active ingredients are co-administered orally.

The active ingredients in the combined pharmaceutical composition disclosed herein can be formulated independently in suitable dosage forms, or some or all of the components are co-formulated in a suitable dosage form including, but not limited to, tablet, lozenge, pill, capsule (for example, hard capsule, soft capsule, enteric capsule and microcapsule), elixir, granule, syrup, injection (intramuscular, intravenous and intraperitoneal), granule, emulsion, suspension, solution, dispersant, and dosage forms of sustained-released preparations for oral or non-oral administration.

### Technical Effects

Generally, use of the combined pharmaceutical composition disclosed herein described above will provide:
(1) better efficacy in controlling tumor growth or even eliminating tumors as compared with either drug of the combination administered alone;
(2) fewer doses as compared with either drug of the combination administered alone;
(3) good tolerability in patients, and fewer adverse effects and/or complications as compared with either drug administered alone;
(4) a higher disease control rate in patients treated;
(5) longer survivals (e.g., median survival time, progression-free survival, or overall survival) in patients treated;
(6) longer survivals (e.g., median survival time, progression-free survival, or overall survival) in patients treated as compared with standard chemotherapies;
(7) a longer duration of response (DOR); and/or
(8) better activity in treating tumors or proliferative diseases and better anti-tumor synergistic effect, as compared with either drug of the combination administered alone.

The "clinical benefits" of the combined pharmaceutical composition disclosed herein include, but are not limited to: prolonged progression-free survival (PFS), prolonged overall survival (OS), improved objective response rate (ORR), improved disease control rate (DCR), reduced number and/or degree of adverse effects, decreased distant metastasis rate, decreased local control rate and the like for clinical patients.

### Definitions and Description

As used herein, the term "combined pharmaceutical composition" refers to a combination of two or more active ingredients (administered as the respective active ingredients themselves, or as their respective derivatives like pharmaceutically acceptable salts or esters, prodrugs, or compositions) that are administered simultaneously or sequentially. As used herein, the terms "combined pharmaceutical composition" and "pharmaceutical combination" are used interchangeably.

The word "comprise", and variants thereof such as "comprises" or "comprising", and equivalents thereof shall be understood in an open, non-exclusive sense, i.e., "includes but is not limited to", indicating that in addition to the listed elements, components and procedures, other unspecified elements, components and procedures may also be encompassed.

The term "patient" or "individual/subject" refers to a mammal, e.g., a primate (human, macaque, chimpanzee, etc.), rodent (mouse, rat, rabbit, etc.), feline, canine, etc., preferably a human. In some embodiments of the present disclosure, the patient and the individual are patients who have failed or lack a standard treatment.

The term "pharmaceutically acceptable" refers to a carrier or an excipient that is used in the preparation of a pharmaceutical composition. The carrier or the excipient is generally safe, non-toxic, and desirable biologically and otherwise, and inclusion of the substance is acceptable for pharmaceutical use in human.

The term "therapeutically effective amount" refers to an amount of a compound that, when administered to a human for treating a disease, is sufficient to treat the disease.

The term "treat", "treating", or "treatment" refers to administering the compound or the formulation described herein so as to ameliorate, alleviate, or eliminate a disease or one or more symptoms associated with the disease, and includes: (i) inhibiting the disease or disease state, i.e., arresting or delaying its development; and (ii) alleviating the disease or disease state, i.e., causing regression of the disease or disease state.

The term "prevent" or "prevention" means administering the compound or formulation described herein to prevent a disease or one or more symptoms associated with the disease, including: preventing the occurrence of the disease or disease state in a mammal, particularly when such a mammal is predisposed to the disease state but has not yet been diagnosed with it.

The term "general treatment" refers to treatment in which a drug substance is transported through the bloodstream to reach and affect cells of the whole body.

The term "systemic treatment" refers to systemic chemotherapy, and systemic or local radiotherapy.

The term "first-line treatment" refers to treatment with drugs that are the first or standard choice according to patient's conditions. As used herein, an "adverse event" (AE) is any adverse and often unintended or undesirable sign (including abnormal laboratory findings), symptom, or disease associated with the use of medical therapy. For example, an adverse event may be associated with activation of the immune system or expansion of immune system cells (e.g., T cells) in response to treatment. The medical treatment may have one or more related AEs, and each AE may have the same or a different severity level. Reference to a method capable of "altering an adverse event" refers to a treatment regimen that reduces the incidence and/or severity of one or more AEs associated with the use of a different treatment regimen.

The use of alternatives (e.g., "or") shall be understood to refer to any one, two, or any combination of the alternatives. As used herein, the indefinite article "a" or "an" shall be understood to mean "one or more" of any listed or enumerated components.

The term "pharmaceutically acceptable excipient" refers to those excipients which do not have a significant irritating effect on an organic entity and do not impair the biological activity and properties of the active compound. Suitable excipients are well known to those skilled in the art, for example, carbohydrate, wax, water-soluble and/or water-swellable polymers, hydrophilic or hydrophobic material, gelatin, oil, solvent, or water.

The terms "administer", "administration" and "administering" refer to physically introducing the composition comprising a therapeutic agent to an individual using any of a variety of methods and delivery systems known to those skilled in the art. In certain embodiments, the administration is oral administration.

The term "daily dose" refers to a dose administered to a patient per day.

The term "single dose" or "unit formulation" refers to the smallest unit of packaging of a pharmaceutical product comprising a certain amount of active ingredients; for example, in a box of seven capsules, each capsule is a single dose or a unit formulation; in a box of seven tablets, each tablet is a single dose or a unit formulation.

The term "multiple dose" consists of multiple single doses. As used herein, "combined use" or "use in combination" means that two or more active substances may be administered to an individual simultaneously, concurrently, or sequentially in any order as a single formulation.

The term "pharmaceutical composition" refers to a mixture consisting of one or more of the active ingredients or pharmaceutical combinations thereof of the present disclosure and a pharmaceutically acceptable excipient. The pharmaceutical composition is intended to facilitate the administration of the compound or the pharmaceutical combination thereof disclosed herein to an individual.

The terms "day", "daily", etc., when referred to in an administration regimen, refer to the time within a calendar day that starts at midnight and ends at the next midnight.

The term "recurrent" cancer is one that regenerates at the initial site or a distant site after being responsive to initial treatment (e.g., surgery). A "locally recurrent" cancer is one that occurs, after treatment, at the same location as the previously treated cancer.

The term "unresectable" cancer is one that cannot be removed by surgery.

The term "metastatic" cancer refers to one that spreads from one part of the body (e.g., the lung) to another part of the body.

As used herein, "combined use" or "use in combination" means that two or more active substances may be administered to a subject as a mixture, simultaneously as a single formulation, or sequentially in any order as a single formulation.

Unless otherwise specified clearly herein, singular terms encompass plural terms, and vice versa. Similarly, unless otherwise specified clearly herein, the word "or" is intended to include "and", and vice versa.

Unless otherwise stated herein, parameter values representing amounts of ingredients or physicochemical properties or reaction conditions and the like are to be understood as being modified in all cases by the term "about". When the term "about" is used to describe the present disclosure, the term "about" indicates that there is an error value; for example, it means varying within a range of ±5%, such as ±1% or ±0.1%, of a particular value. All patents, patent applications and other identified publications are explicitly incorporated herein by reference for the purpose of description and disclosure. These publications are provided solely because they were disclosed prior to the filing date of the present disclosure. All statements as to the dates of these documents or description as to the contents of these documents are based on the information available to the applicant and do not constitute any admission as to the correctness of the dates or the content of these documents. Moreover, in any country or region, any reference to these publications herein is not to be construed as an admission that the publications form part of the commonly recognized knowledge in the art.

### Example

The following specific examples are intended to allow those skilled in the art to clearly understand and implement the present disclosure. These specific examples should not be considered as limit to the scope of the present disclosure, but merely as exemplary description and representative of the present disclosure.

### Experimental Example 1. Clinical Trial

The study was divided into 2 cohorts, namely cohort I and cohort II, and the study drugs are the compound of formula (I) and fulvestrant injection in combination for both cohorts. Subjects with HR-positive and HER2-negative locally advanced and/or metastatic breast cancer were enrolled, and 30-60 subjects were included in each cohort. The preliminary efficacy and safety of the compound of formula (I) in combination with fulvestrant injection were evaluated.
1.1. Inclusion criteria:
   1) Voluntary participation, written informed consent, and good compliance;
   2) Aged 18-75 years (when signing the informed consent); ECOG PS score: 0-1; expected survival time of more than 3 months;
   3) Subjects with locally advanced or metastatic breast cancer, where the primary or metastatic tumor was HR-positive and HER2-negative as confirmed by pathological examination.
   4) Subjects enrolled in cohort I in the recurrent/metastatic stage were allowed to receive therapy no more than first-line;
   5) Subjects enrolled in cohort II had not previously received systemic anti-tumor therapy;
   6) It was confirmed that there was at least one measurable lesion according to RECIST 1.1;
   7) Good main organ functions meeting the following criteria:
      Blood routine examination criteria (no blood transfusion and no correction using hematopoietic stimulating drugs within the last 7 days before screening):
         a) Hemoglobin (HB) ≥ 100 g/L;
         b) Absolute value of neutrophil count (NEUT) ≥ 1.5 × 10⁹/L;
         c) Platelet count (PLT) ≥ 90 × 10⁹/L.
      Biochemical test results should meet the following criteria:
         a) Total bilirubin (TBIL) ≤ 2.5 times of upper limit of normal (ULN);
         b) Alanine aminotransferase (ALT) and aspartate aminotransferase (AST) ≤ 2.5 × ULN. If liver metastasis is accompanied, ALT and AST ≤ 5 × ULN;
         c) Serum creatinine (CR) ≤ 1.5 × ULN or creatinine clearance rate (CCR) ≥ 60 mL/min.
      Blood coagulation function examination should meet the following criteria:
         Prothrombin time (PT), activated partial thromboplastin time (APTT), and international normalized ratio (INR) ≤ 1.5 × ULN (not previously received anticoagulant therapy);
         Heart color ultrasound evaluation: left ventricular ejection fraction (LVEF) ≥ 50%.
1.2 Test drug
   Capsules of the compound of formula (I): strength: 50 mg or 60 mg, provided by Chia Tai Tianqing Pharmaceutical Group Co., Ltd.
   Fulvestrant injection: strength: 250 mg, provided by Chia Tai Tianqing Pharmaceutical Group Co., Ltd.
1.3. Administration regimen
   Capsules of the compound of formula (I): 180 mg/dose (based on the compound of formula (I)), oral administration at fasting, once daily, and 28 consecutive days of administration as one treatment cycle. Fulvestrant injection: 500 mg/dose of intramuscular injection; every 28 days were counted as one treatment cycle; administered on day 1 and day 15 in the first treatment cycle and on day 1 in each subsequent treatment cycle.
1.4. Evaluation criteria
   Effectiveness evaluation criteria: disease status was determined using the RECIST 1.1 criteria.
   Safety evaluation criteria: the severity of adverse events was determined using the NCI-CTC AE 5.0 criteria. Adverse event record forms including time of occurrence, severity, relevance to the studied treatment, duration, measures taken, and metastasis and progression should be faithfully filled out during the trial.
1.5 Results of trial
   1.5.1 Safety
      Gastrointestinal reactions (diarrhea, vomiting, and the like) were mainly grade 1-2, and could be controlled after symptomatic treatment. The total incidence rate of TEAEs of grade 3 or more was 39.2%, the incidence rate of hematologic toxicity of grade 3 was 9.6%, the incidence rate of diarrhea of grade 3 was 7.8%, and the overall safety was good.
   1.5.2 Efficacy
      A total of 110 patients were enrolled, and the median duration of treatment for the evaluable patients was 6.6 months. The objective response rate (ORR) was 58.2% (64/110), including 2 patients with complete response (CR), 62 patients with partial response (PR), and 38 patients with stable disease (SD), and the disease control rate (DCR) was 92.7% (102/110). The results showed that the combined pharmaceutical composition of the present disclosure had clinical benefits.

### Representative cases:

| Subject No. | History of previous therapy | Dosage regimen | Administration cycle | Efficacy evaluation | | | | | | |
|---|---|---|---|---|---|---|---|---|---|---|
| | | | | C2 | C4 | C6 | C8 | C10 | C12 | C14 |
| 1 | 1. TP-4 cycles; | Capsules of the compound of formula (I): 180 mg/dose, once daily; 28 days as one treatment cycle. Fulvestrant injection: 500 mg/dose, intramuscular injection; administered on day 1 and day 15 in the first treatment cycle and on day 1 in each subsequent treatment cycle; 28 days as one treatment cycle. | C6 | PR | PR | PR | -- | -- | -- | -- |
| | 2. Modified radical mastectomy for right-breast cancer; | | | | | | | | | |
| | 3. TP-4 cycles; | | | | | | | | | |
| | 4. Radiotherapy; | | | | | | | | | |
| | 5. Goserelin therapy; | | | | | | | | | |
| | 6. Tamoxifen therapy; | | | | | | | | | |
| 2 | 1. Modified radical mastectomy for right-breast cancer; | Same as No. 1 | C8 | PR | PR | PR | PR | -- | -- | -- |
| | 2. TAC-1 cycle; | | | | | | | | | |
| 3 | 1. Modified radical mastectomy for right-breast cancer; | Same as No. 1 | C8 | PR | PR | PR | PR | -- | -- | -- |
| | 2. Modified radical mastectomy for left-breast cancer; | | | | | | | | | |
| | 3. TAC-6 cycles; | | | | | | | | | |
| | 4. Tamoxifen; | | | | | | | | | |
| | 5. Metastasis and radiotherapy; | | | | | | | | | |
| 4 | Treatment-naive patient with stage IV primary cancer | Same as No. 1 | C10 | SD | SD | SD | PR | PR | -- | -- |
| 5 | Treatment-naive patient with stage IV primary cancer | Same as No. 1 | C6 | PR | PR | PR | -- | -- | -- | -- |
| 6 | 1. Modified radical mastectomy for right-breast cancer; | Same as No. 1 | C8 | CR | CR | CR | CR | | | |
| | 2. R-CDOP-6 cycles; | | | | | | | | | |
| | 3. Chemotherapy-7 cycles; | | | | | | | | | |
| | 4. Tamoxifen; | | | | | | | | | |

| | | | | | | | | | | |
|---|---|---|---|---|---|---|---|---|---|---|
| Note: "--" indicates that the data has not been obtained yet. | | | | | | | | | | |

Those skilled in the art will recognize that the scope of the present disclosure is not limited to the various embodiments and examples described above. Instead, various modifications, substitutions, or recombinations can be made without departing from the spirit and conception of the present disclosure, all of which fall within the protection scope of the present disclosure.

## Claims

1. A combined pharmaceutical composition comprising a compound of formula (I) or a pharmaceutically acceptable salt thereof and fulvestrant

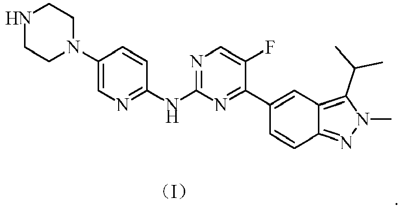

2. The combined pharmaceutical composition according to claim 1, comprising a pharmaceutical composition of the compound of formula (I) or the pharmaceutically acceptable salt thereof, and a pharmaceutical composition of fulvestrant.

3. The combined pharmaceutical composition according to claim 1 or 2, comprising 20-240 mg, 40-180 mg, 60-180 mg, 80-180 mg, 100-180 mg, 120-180 mg, or 150-180 mg of the compound of formula (I) or the pharmaceutically acceptable salt thereof based on the compound of formula (I), or a pharmaceutical composition thereof; or comprising 20 mg, 40 mg, 60 mg, 80 mg, 100 mg, 120 mg, 150 mg, 180 mg, or 240 mg of the compound of formula (I) or the pharmaceutically acceptable salt thereof based on the compound of formula (I), or a pharmaceutical composition thereof.

4. The combined pharmaceutical composition according to claim 3, wherein the pharmaceutical composition of the compound of formula (I) or the pharmaceutically acceptable salt thereof is in a multi-dose form.

5. The combined pharmaceutical composition according to any of claims 1-4, comprising 125-1000 mg, 125-750 mg, 250-750 mg, or 250-500 mg of fulvestrant, or a pharmaceutical composition thereof; or comprising 125 mg, 250 mg, 500 mg, 750 mg, or 1000 mg of fulvestrant, or a pharmaceutical composition thereof.

6. The combined pharmaceutical composition according to claim 5, wherein the pharmaceutical composition of fulvestrant is in a multi-dose form.

7. The combined pharmaceutical composition according to any of claims 1-6, comprising 120-180 mg of the compound of formula (I) or the pharmaceutically acceptable salt thereof based on the compound of formula (I), and 250-500 mg of fulvestrant.

8. The combined pharmaceutical composition according to claim 1 or 2, wherein the compound of formula (I) or the pharmaceutically acceptable salt thereof is prepared to comprise 5 mg, 50 mg, or 60 mg of the compound of formula (I) or the pharmaceutically acceptable salt thereof based on the compound of formula (I) in a unit formulation, and the fulvestrant is prepared to comprise 250 mg of fulvestrant in a unit formulation.

9. The combined pharmaceutical composition according to claim 1 or 2, wherein the combined pharmaceutical composition is a formulation suitable for administration within a single treatment cycle, and the formulation comprises a pharmaceutical composition comprising the compound of formula (I) or the pharmaceutically acceptable salt thereof in a total dose of 1680-5040 mg based on the compound of formula (I), and a pharmaceutical composition comprising fulvestrant in a total dose of 500-1000 mg based on fulvestrant.

10. The combined pharmaceutical composition according to claim 1 or 2, wherein the combined pharmaceutical composition is a formulation suitable for administration within a single treatment cycle, and the formulation comprises a pharmaceutical composition comprising the compound of formula (I) or the pharmaceutically acceptable salt thereof and a pharmaceutical composition comprising fulvestrant in a total dose ratio of (1-30): 1, wherein the dose of the pharmaceutical composition comprising the compound of formula (I) or the pharmaceutically acceptable salt thereof is based on the compound of formula (I), and the dose of the pharmaceutical composition comprising fulvestrant is based on fulvestrant.

11. The combined pharmaceutical composition according to any of claims 1-10, wherein a content of the compound of formula (I) or the pharmaceutically acceptable salt thereof is a dose per treatment cycle, which is administered by administering the compound of formula (I) or the pharmaceutically acceptable salt thereof daily; a content of fulvestrant is a dose per treatment cycle, which is administered by administering fulvestrant on day 1 and day 15 in the first treatment cycle, and on day 1 in each subsequent treatment cycle.

12. Use of the combined pharmaceutical composition according to any of claims 1-11 for preparing a medicament for use in treating or preventing breast cancer.

13. A combined pharmaceutical composition according to any of claims 1-11 for use in treating or preventing breast cancer.

14. A method of treating or preventing breast cancer, comprising administering to an individual in need thereof a therapeutically effective amount of the combined pharmaceutical composition according to any of claims 1-11.

15. The use according to claim 12, the combined pharmaceutical composition for use according to claim 13, or the method according to claim 14, wherein the breast cancer is selected from the group consisting of:
HR-positive and HER2-negative breast cancer;
HR-positive and HER2-negative locally advanced and/or metastatic breast cancer;
HR-positive and HER2-negative locally advanced and/or metastatic breast cancer unable to receive radical surgery or radiotherapy; or
postmenopausal or premenopausal/perimenopausal HR-positive and HER2-negative locally advanced and/or metastatic breast cancer.
